# EUROPÄISCHE PATENTSCHRIFT

(11) **EP 0 452 637 B1**
(45) Veröffentlichungstag und Bekanntmachung des Hinweises auf die Patenterteilung: **25.09.1996**
(21) Anmeldenummer: 91102780.3
(22) Anmeldetag: 26.02.1991
(51) Int. Cl.: G21K 5/02

(54) **Bestrahlungsvorrichtung**
Irradiation device
Dispositif d'irradiation

(30) Priorität: 19.04.1990 DE 4012398
(43) Veröffentlichungstag der Anmeldung: 23.10.1991
(73) Patentinhaber: Wälischmiller, Hans, D-88677 Markdorf (DE)
(72) Erfinder: Wälischmiller, Hans, D-88677 Markdorf (DE)
(74) Vertreter: Engelhardt, Guido, Dipl.-Ing.

(56) Entgegenhaltungen:
- FR-A- 2 020 234
- FR-A- 2 443 122
- FR-A- 2 526 991
- US-A- 2 872 587

## Beschreibung

Die Erfindung bezieht sich auf eine Vorrichtung zum Bestrahlen eines Gegenstandes, insbesondere eines in einem Behälter gelagerten flüssigen oder gasförmigen Mediums, wie beispielsweise Blut, mit einer Strahlungsquelle für ionisierende Strahlen, die in einem abgeschirmten Gehäuse eingesetzt ist und in diesem auf das zu bestrahlende Medium einwirkt.

Bei einer bekannten Bestrahlungsvorrichtung dieser Art ist das die Strahlungsquelle aufnehmende Gehäuse in deren Höhe seitlich mit einer offenen Ausnehmung versehen, in dem ein Gehäuseteil verdrehbar gelagert ist, das eine Bestrahlungskammer aufweist. Der zu bestrahlende Gegenstand, z.B. eine Flasche, wird bei dieser Ausgestaltung in die Bestrahlungskammer eingesetzt und kann durch Verdrehen des Gehäuses zur Strahlungsquelle transportiert werden. Da der Gegenstand mittels eines in der Bestrahlungskammer vorgesehenen Drehtellers des weiteren um seine Achse gedreht werden kann, ist zwar eine gleichmäßige Bestrahlung von außen möglich, trotz des erheblichen Bauaufwandes können aber nur in einer Flasche oder in einem Beutel eingefüllte und somit ruhende Medien jeweils von einer Seite der Strahlungsquelle bestrahlt werden. Außerdem ist der Zeitaufwand dazu, da das Behältnis und somit das zu bestrahlende Medium an der Strahlungsquelle vorbeizubewegen ist, erheblich, auch kann sich die Rotationsbewegung des Behältnisses ungünstig auf das zu bestrahlende Medium auswirken. Der Anwendungsbereich dieser Bestrahlungsvorrichtung ist daher begrenzt, da diese insbesondere bei einer Operation, um z.B. während dieser das Blut eines Patienten zu bestrahlen, nicht eingesetzt werden kann.

Aufgabe der Erfindung ist es demnach, eine Bestrahlungsvorrichtung der vorgenannten Gattung zu schaffen, mittels der nicht nur eine äußerst intensive und gefahrlose Bestrahlung eines Mediums vorzunehmen ist, sondern auch ein fließendes Medium nahe an der Strahlungsquelle vorbeiströmen kann, wobei die auf dieses einwirkende Strahlendosis leicht den Erfordernissen, beispielsweise in Abhängigkeit von der Strömungsgeschwindigkeit, entsprechend einzustellen ist. Ferner soll die Bestückung der Bestrahlungsvorrichtung ohne Schwierigkeiten vorzunehmen und somit soll eine einfache Handhabung gegeben sein, dennoch soll zuverlässig gewährleistet werden, daß keine Strahlen unkontrolliert nach außen gelangen und daß die Bestrahlungsvorrichtung folgerichtig zu bedienen und entsprechend funktionsfähig ist.

Gemäß der Erfindung wird dies dadurch erreicht, daß das vorzugsweise in Form einer Tonne ausgebildete Gehäuse auf der einen Seite eine zentrische Aufnahmekammer für die Strahlungsquelle und fluchtend zu dieser auf der anderen Seite eine Bestrahlungskammer zur Aufnahme eines das zu bestrahlende Medium enthaltende Behältnisses aufweist, dass zwischen der Aufnahmekammer und der Bestrahlungskammer ein z.B. verdrehbar oder verschiebbar gelagerter Schieber angeordnet ist, der mit einer Durchgangsöffnung für die in die Bestrahlungskammer einführbare Strahlungsquelle versehen und in unterschiedlichen Winkelstellungen arretierbar ist, und daß die Bestrahlungskammer durch einen zustellbaren Verschlußstopfen verschließbar ist.

Zweckmäßig ist es hierbei, das das zu bestrahlende Medium aufnehmende Behältnis mit einer zentrisch angeordneten Ausnehmung zur Aufnahme der Strahlungsquelle zu versehen, wobei dieses durch einen schraubenlinienförmig um eine die Strahlungsquelle aufnehmenden hohlen Kern gewickelten Schlauch gebildet oder aus zwei mit Abstand ineinander angeordneten Rohren bestehen kann, deren Ringraum durch Zwischenwände in miteinander kommunizierende Strömungskanäle unterteilt ist. Um den Transport des Mediums durch das Behältnis sicherzustellen, sollte dieses an eine Pumpvorrichtung, beispielsweise eine an der Bestrahlungsvorrichtung angebrachte Schlauchpumpe, anschließbar sein.

Vorteilhaft ist es des weiteren, wenn der Schieber mittels eines Hebelgestänges in drei Betriebsstellungen verstellbar und in diesen arretierbar ist.

Das dazu verwendbare Hebelgestänge kann in einfacher Weise aus einem außerhalb des Gehäuses angeordneten drehbar gelagerten Betätigungshebel, einem an dem Schieber angebrachten Verstellhebel und einem an diesen angelenkten Verbindungshebel bestehen, und dem Betätigungshebel sollte ein in Abhängigkeit von der Betriebsstellung des Verschlußstopfens lösbares Verriegelungsglied zugeordnet sein.

Zweckmäßig ist es ferner, die Strahlungsquelle mittels eines vorzugsweise motorisch antreibbaren Hubgliedes in die Bestrahlungskammer des Gehäuses einzuführen und den Verschlußstopfen mit Hilfe einer Brücke an einem aus zwei Führungsschienen bestehenden Traggestell ebenfalls mittels eines Motors in der Höhe zu verstellen.

Angebracht ist es auch, die Brücke mit einem Schaltnocken auszustatten, mittels dem das Verriegelungsglied des dem Schieber zugeordneten Hebelgestänges lösbar ist, und das das zu bestrahlende Medium aufnehmende Behältnis an dem Verschlußstopfen, beispielsweise mittels einer gestuft ausgebildeten Schraube, lösbar zu befestigen.

Des weiteren sollte der Verschlußstopfen in eine fluchtend zu der Aufnahmekammer des Behältnisses in das Gehäuse eingearbeitete, erweiterte Aufnahmeöffnung einsetzbar, mit einer die Aufnahmeöffnung übergreifenden Abdeckung und vorzugsweise in seinem Randbereich mit einer Freisparung zur Durchführung von an das Behältnis anzuschließenden Leitungen versehen sein.

Das Gehäuse sollte aus fertigungstechnischen Gründen aus einem die Strahlungsquelle aufnehmenden Unterteil, einem Zwischenstück zur verdrehbaren Lagerung des Schiebers und einem das Behältnis aufnehmenden Oberteil zusammengesetzt sein. Ferner kann das Gehäuse mit einer geschlossenen Ummantelung versehen werden, an der auch eine Anzeigeeinrichtung angebracht sein kann, die mechanisch unmittelbar mit der Strahlungsquelle oder deren Hubglied verbunden ist. Wird eine Bestrahlungsvorrichtung gemäß der Erfindung ausgebildet, so ist es ohne Schwierigkeiten möglich, den zu bestrahlenden Gegenstand äußerst intensiv den von der Strahlungsquelle abgegebenen ionisierenden Strahlen auszusetzen, aber dennoch gefahrlos zu bestrahlen; vor allem aber kann auch ein fliessendes Medium problemlos bestrahlt werden. Wird nämlich das Gehäuse der Bestrahlungsvorrichtung mit einer Aufnahmekammer versehen, in die das zu bestrahlende Medium aufnehmende in besonderer Weise gestaltete Behältnis einsetzbar ist und wird die Strahlungsquelle derart angeordnet, daß diese in das Aufnahmebehältnis eingeführt werden kann, wobei mittels eines gesteuert zu betätigenden Schiebers nur in einer vorgegebenen Betriebsstellung die Einführung zu bewerkstelligen ist, so wirken die Strahlen von innen auf das die Strahlungsquelle einschliessende Medium ein. Die Strahlungsintensität wird somit sehr gut ausgenutzt, so daß die Bestrahlungszeit bei Übertragung der gleichen Strahlendosis erheblich kürzer ist als bei der bekannten Vorrichtung.

Auch wirken bei der vorschlagsgemäßen Bestrahlungsvorrichtung auf das zu bestrahlende Medium, da das dieses aufnehmende Behältnis während einer Bestrahlung stationär angeordnet ist, keine Zentrifugalkräfte, die u. U. zu Abscheidungen führen können, ein, des weiteren ist die Bestrahlung beispielsweise in Abhängigkeit von der Stellung der Strahlungsquelle, der Bestrahlungsdauer und/oder der Fließgeschwindigkeit des Mediums leicht zu dosieren, gegebenenfalls auch zu programmieren. Insbesondere aber ist von Vorteil, daß ein Medium das Behältnis während einer Bestrahlung durchströmen kann, das Bestrahlungsgerät kann somit auch unmittelbar an einen Patienten angeschlossen oder während einer Operation eingesetzt werden. Aufgrund der einfachen Handhabung und der hohen Betriebssicherheit ist dabei gewährleistet, daß der Einsatz sowohl für das Personal als auch für einen Patienten stets gefahrlos ist. Und da der Behälter und dessen Zuleitung rasch ausgetauscht und steril gehaltenwerden können, ist, zumal auch die Bestrahlungzeit kurz ist, ein hoher Ausnutzungsgrad gegeben. Durch die Anordnung eines nur bei abgeschirmter Bestrahlungskammer zu öffnenden Schiebers zwischen dieser und der Aufnahmekammer der Stahlungsquelle ist ferner gewährleistet, daß eine unkontrollierte Abstrahlung und eine Gefährdung des Personals ausgeschlossen ist.

In der Zeichnung ist ein Ausführungsbeispiel der gemäß der Erfindung ausgebildeten Bestrahlungsvorrichtung dargestellt, das nachfolgend im einzelnen erläutert ist. Hierbei zeigt:
- Figur 1: die Bestrahlungsvorrichtung während eines Bestückungsvorganges in einem Axialschnitt,
- Figur 2: die Vorrichtung nach Figur 1 in Seitenansicht während einer Bestrahlung,
- Figuren 3 bis 5: die unterschiedlichen Betriebsstellungen des mittels eines Hebelgestänge betätigbaren Schiebers, der Bestrahlungsvorrichtung nach den Figuren 1 und 2,
- Figur 6: das bei der Bestrahlungsvorrichtung nach den Figuren 1 und 2 vorgesehene Behältnis zur Aufnahme eines zu bestrahlenden flüssigen Mediums,
- Figur 7: eine andersartige Ausgestaltung eines derartigen Behältnisses in einem Axialschnitt und
- Figur 8: das Behältnis nach Figur 7 in Draufsicht.

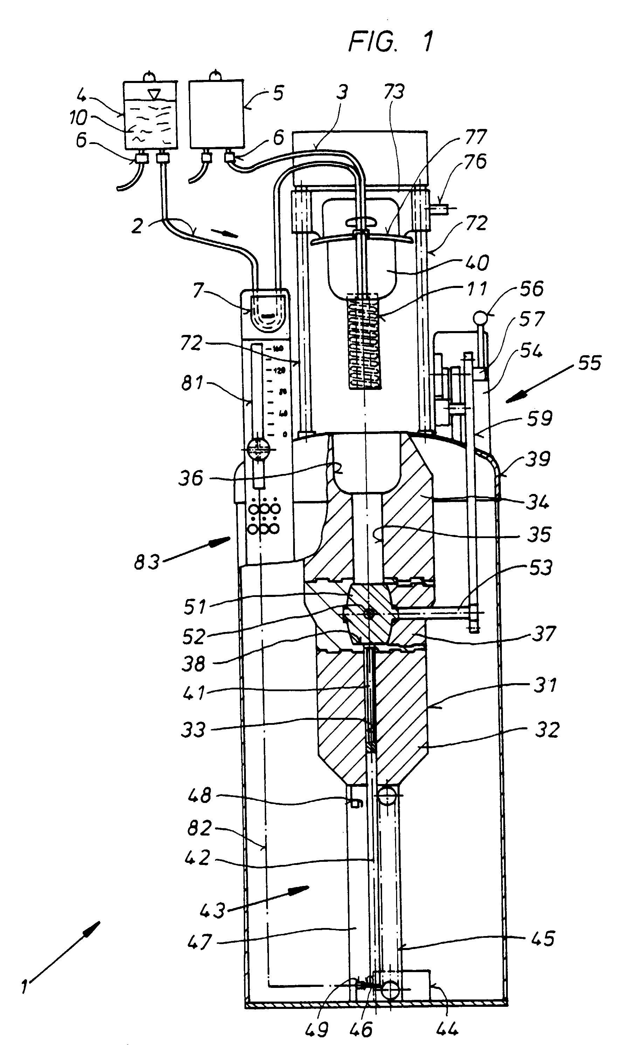

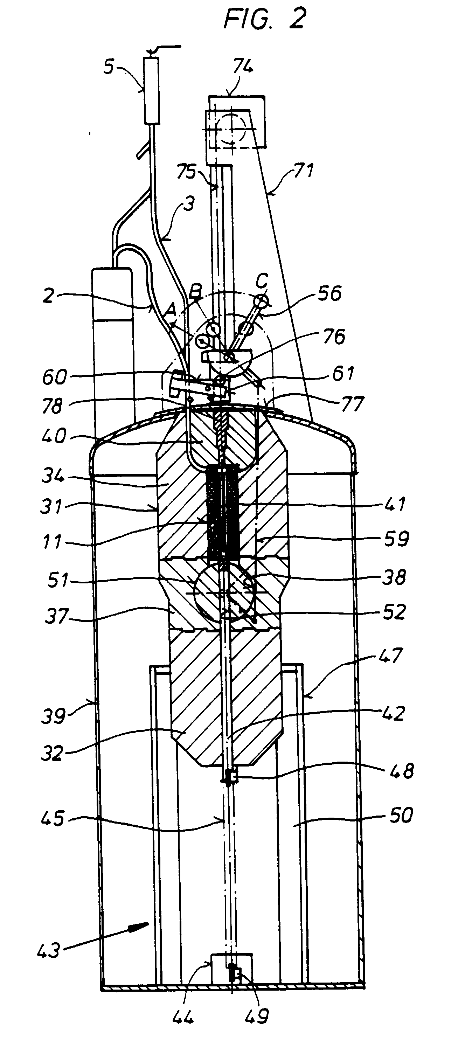

Die in den Figuren 1 und 2 dargestellte und mit 1 bezeichnete Vorrichtung dient zum Bestrahlen eines Gegenstandes, vorzugsweise eines flüssigen Mediums 10 mit ionisierenden Strahlen, das über eine Zuführungsleitung 2 einem Behältnis 11 zuströmen kann, das in die Vorrichtung 1 einsetzbar ist. Dazu sind in einem tonnenförmig ausgebildeten Gehäuse 31 eine Aufnahmekammer 33 für eine Strahlungsquelle 41 sowie eine Bestrahlungskammer 35 eingearbeitet, in die die Strahlungsquelle 41 einführbar ist. Zwischen der Aufnahmekammer 33 und der Bestrahlungskammer 35 ist des weiteren ein mittels eines Hebelgestänges 55 gesteuert verstellbarer Schieber 51 angeordnet, durch den die Aufnahmekammer 33 der Strahlungsquelle 41 bei einer Beschickung der Vorrichtung 1, wie dies in Figur 1 dargestellt ist, abgeschirmt ist.

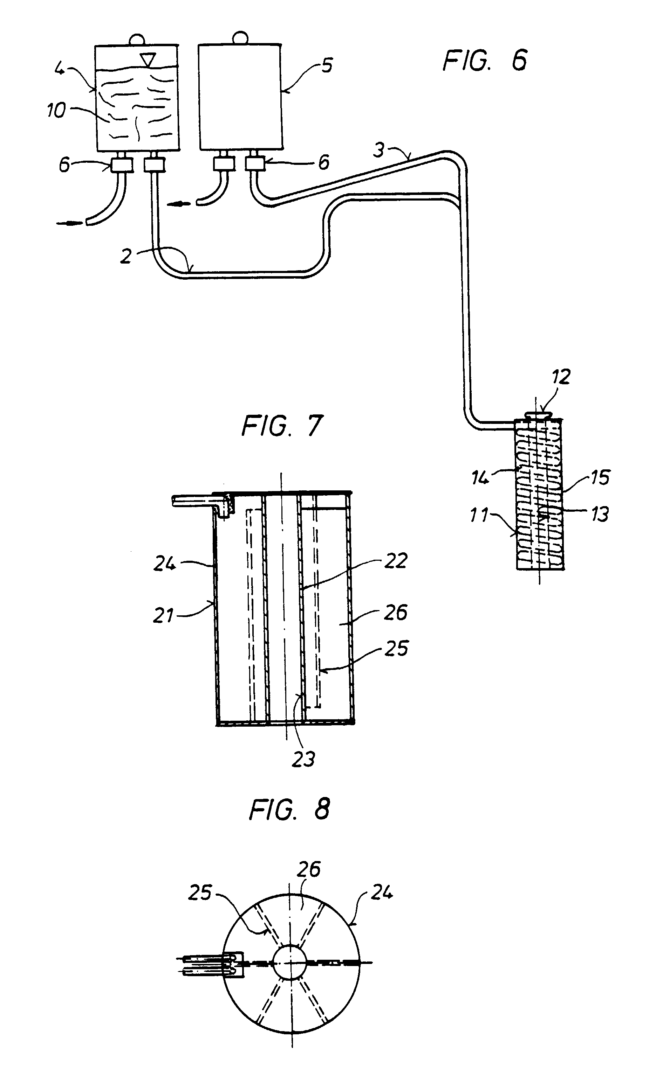

Der Behälter 11 zur Aufnahme der zu bestrahlenden Flüssigkeit 10 besteht, wie dies im einzelnen der Figur 6 zu entnehmen ist, aus einem auf einem hohlen Kern 12 schraubenlinienförmig aufgewickelten Schlauch 14 und einer Ummantelung 15, so daß eine Öffnung 13 gebildet ist, in die die Strahlungsquelle 41 eingeführt werden kann. Gemäß den Figuren 7 und 8 kann der dort dargestellte Behälter 21 aber auch aus einem Innenrohr 22 und einem mit Abstand zu diesem angeordneten Außenrohr 24 hergestellt werden, zwischen denen Zwischenwände 25 derart eingesetzt sind, daß miteinander kommunizierende Strömungskanäle 26 geschaffen sind. Die Strahlungsquelle 41 kann bei dieser Ausgestaltung während eines Bestrahlungsvorganges in die Öffnung 23 des Innenrohres 22 eingeführt werden.

Über die Zuführungsleitung 2 sowie eine Rückführungsleitung 3 kann das Behältnis 11 bzw. 21 unmittelbar an einem Patienten, um z.B. dessen Blut zu bestrahlen, angeschlossen weraen, es ist aber auch möglich, an die mit Absperrventilen 6 ausgestatteten Leitungen 2 und 3 Blutbeutel 4 und 5 anzuschließen, in denen die zu bestrahlende Flüssigkeit 10 zwischengelagert wird. Mittels einer an der Bestrahlungsvorrichtung 1 angebrachten Schlauchpumpe 7 wird die zu bestrahlende Flüssigkeit 10 dem Behältnis 11 bzw. 21 während eines Bestrahlungsvorganges kontinuierlich zugeführt.

Das von einer Ummantelung 39 umgebene Gehäuse 31 der Bestrahlungsvorrichtung 1 ist aus einem Unterteil 32, in dem die Aufnahmekammer 33 der Strahlungsquelle 41 vorgesehen ist, einem die Bestrahlungskammer 35 aufweisenden Oberteil 34 und einem Zwischenstück 37 zur verdrehbaren Lagerung des Schiebers 51 in einer in dieses eingearbeiteten Ausnehmung 38 zusammengesetzt. Das Oberteil 34 ist des weiteren mit einer gegenüber der Bestrahlungskammer 35 erweiterten Aufnahmeöffnung 36 für einen Verschlußstopfen 40 ausgestattet. Mittels Stützen 50 ist das Gehäuse 31 gehalten.

Zur Verstellung der in die Bestrahlungskammer 35 einführbaren und somit höhenverstellbar angeordneten Strahlungsquelle 41 dient ein auf Führungsschienen 47 abgestütztes Hubglied 43 in Form eines durch einen Motor 44 antreibbaren Kettentriebes 45, an dem eine Platte 46 angebracht ist. Und die Strahlungsquelle 41 ist auf einer aus dem Unterteil 32 des Gehäuses 31 ragenden Stange 42 angeordnet, die auf einer Platte 46 ruht. Die Stange 42 dient auch zur Abschirmung der Aufnahmekammer 33. Durch eine Verdrehung des Kettentriebes 45 im Uhrzeigersinn kann somit die Strahlungsquelle 41 angehoben und bei entsprechender Stellung des Schiebers 51 ganz oder teilweise, je nach Dosierung in die Bestrahlungskammer 35 eingeführt werden. Die Verstellbewegungen des Hubgliedes 43 werden mittels Endschalter 48 und 49 überwacht.

Damit die Strahlungsquelle 41 in einer bestimmten Stellung des Schiebers 51 in die Bestrahlungskammer 35 eingeschoben werden kann, ist dieser mit einer Durchgangsbohrung 52 versehen. Die Verstellung des Schiebers 51 ist mittels des Hebelgestänges 55 zu bewerkstelligen, das auf eine an einer an dem Schieber 51 angebrachten Welle 53 einwirkt.

Das Hebelgestänge 55 besteht hierbei aus einem mittels einer Welle 57 drehbar in einer Konsole 54 gelagerten Betätigungshebel 56, einem mit der Welle 53 des Schiebers 51 verbundenen Verstellhebel 58 sowie einem Verbindungshebel 59, der an dem Betätigungshebel 56 sowie dem Verstellhebel 58 angelenkt ist. Der Betätigungshebel 56 ist des weiteren mit einem Riegelglied 60 ausgestattet, das mit einem um einen Bolzen 62 entgegen der Kraft einer Feder 63 verstellbaren Anschlag 61 zusammenwirkt. Mittels eines Riegels 64, der an dem Betätigungshebel 56 angebracht und in die Konsole 54 eingearbeitete Ausnehmungen 65, 66 oder 67 einführbar ist, ist der Betätigungshebel 56 in den Stellungen A, B oder C arretierbar.

Der Verschlußstopfen 40 ist mittels einer Brücke 73 höhenverstellbar an einem Traggestell 71 gehalten, das dazu zwei Führungsschienen 72 aufweist. Und an der Brücke 73 ist eine aufwickelbare Kette 75 befestigt, die mit Hilfe eines Motors 74 verstellbar ist. Außerdem ist an der Brücke 73 ein Verstellnocken 76 angebracht, der mit dem Riegelglied 60 des Betätigungshebels 56 zusammenwirkt. Mittels einer abgestuft ausgebildeten Schraube 78 ist das Behältnis 11 bzw. 21 abnehmbar an dem Verschlußstopfen 40, an dem eine die Aufnahmeöffnung 36 übergreifende Abdeckung 77 angebracht ist, befestigt. Und um die Zuführungsleitung 2 sowie die Rückführungsleitung 3 bei in die Bestrahlungsvorrichtung 1 eingesetztem Behältnis 11 bzw. 21 aus der Bestrahlungskammer 35 herausführen zu können, ist der Verschlußstopfen 40 im Randbereich mit einer entsprechenden Freisparung 68 versehen.

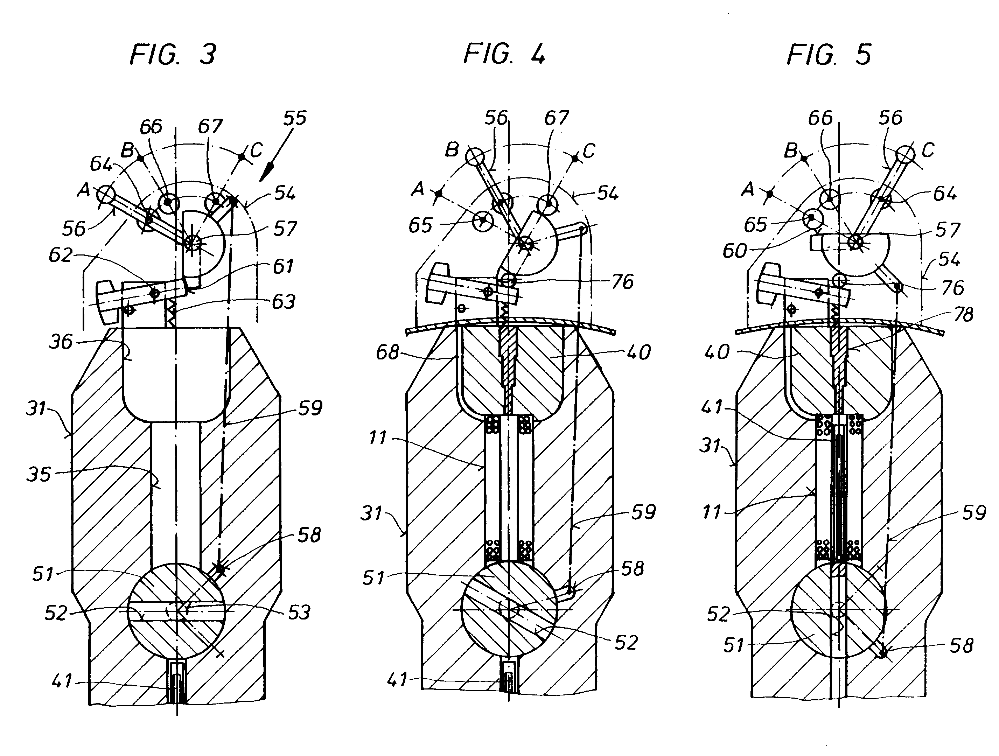

Soll ein Behältnis 11 bzw. 21 in die Bestrahlungsvorrichtung 1 eingesetzt werden, so ist der Verschlußstopfen 40 in die in Figur 1 gezeigte Stellung zu bringen. Vorher ist der Schieber 51 jedoch derart zu verdrehen - wie dies in Figur 3 dargestellt ist -, daß die in diesen eingearbeitete Durchgangsbohrung 52 nicht mit der Aufnahmekammer 33 fluchtet, damit die in dieser befindliche Strahlungsquelle 41 auch abgeschirmt ist. Das an dem Betätigungshebel 56 angebrachte Riegelglied 60 liegt in Position A des Betätigungshebels 56 an dem Anschlag 61 an, eine unabsichtliche Verstellung des Schiebers 51 ist somit ausgeschlossen.

Sodann wird das Behältnis 11 bzw. 21 an dem Verschlußstopfen 40 festgeschraubt und dieser wird in die Aufnahmeöffnung 36 eingesetzt. Dabei wird mittels des an der Brücke 73 angebrachten Schaltnockens 76 der Anschlag 61 entgegen der Kraft der auf diesen einwirkenden Feder 63 verstellt, der Betätigungshebel 56 kann somit in Position B gemäß Figur 4 verstellt werden. In diesem Betriebszustand befindet sich das Behältnis 11 bzw. 21 bereits in der Bestrahlungskammer 35, die Strahlungsquelle 41 kann aber noch nicht in diese eingeführt werden, da die Druchgangsbohrung 52 des Schiebers 51 nicht mit der Aufnahmekammer 33 fluchtet.

In dieser Betriebsstellung kann die Bestrahlungsvorrichtung 1 beispielsweise in einen Operationssaal gebracht oder das Behältnis 11 bzw. 21 kann an einen Patienten angeschlossen werden, ohne daß sofort eine Bestrahlung vorgenommen wird.

Wird allerdings der Betätigungshebel 56 in Position C weitergedreht, so wird dadurch der Schieber 51 über das Hebelgestänge 55, wie dies in Figur 5 gezeigt ist, derart verstellt, daß die Strahlungsquelle 41 mit Hilfe des Hubgliedes 43 mehr oder weniger in die Öffnung 13 bzw. 23 des Behältnisses 11 bzw. 21 eingeführt werden kann und daß das in diesem befindliche oder durch diese strömende Medium bestrahlt wird. In Abhängigkeit von der Durchflußgeschwindigkeit des Mediums, der Stellung der Strahlungsquelle 41 und/oder der Zeitdauer der Bestrahlung kann hierbei die Strahlendosis, die dem Medium 10 zugeführt wird, geregelt werden.

In dem Riegel 64 kann ein Schließzylinder eingebaut sein, damit der Betätigungshebel 56 in den Positionen A, B oder C gegen unbeabsichtigtes Verdrehen zu sichern ist. Auch kann, wie dies in Figur 1 strichpunktiert eingezeichnet ist, die Strahlungsquelle 41 oder das dieser zugeordnete Hubglied 43 über Verbindungsglieder 82 mechanisch mit einer an der Ummantelung 39 angebrachten Anzeigeeinrichtung 81 verbunden sein. Auf diese Weise kann somit die Lage der Strahlungsquelle 41 jederzeit bestimmt werden. Des weiteren können an der Ummantelung 39 Kontroll- oder Anzeigeelemente 83 oder ein Rechner, mit dem ein automatischer Ablauf einer Behandlung zu bewerkstelligen ist und/oder die Bestrahlungswerte zu speichern sind, vorgesehen sein.

## Patentansprüche

1. Vorrichtung zum Bestrahlen eines Gegenstandes, insbesondere eines in einem Behälter gelagerten flüssigen oder gasförmigen Mediums, wie beispielsweise Blut, mit einer Strahlungsquelle für ionisierende Strahlen, die in einem abgeschirmten Gehäuse eingesetzt ist und in diesem auf das zu bestrahlende Medium einwirkt,
**dadurch gekennzeichnet**,
daß das vorzugsweise in Form einer Tonne ausgebildete Gehäuse (31) auf der einen Seite eine zentrische Aufnahmekammer (33) für die Strahlungsquelle (41) und fluchtend zu dieser auf der anderen Seite eine Bestrahlungskammer (35) zur Aufnahme eines das zu bestrahlende Medium (10) enthaltende Behältnisses (11; 21) aufweist, daß zwischen der Aufnahmekammer (33) und der Bestrahlungskammer (35) ein z.B. verdrehbar oder verschiebbar gelagerter Schieber (51) angeordnet ist, der mit einer Durchgangsöffnung (52) für die in die Bestrahlungskammer (35) einführbare Strahlungsquelle (41) versehen und in unterschiedlichen Winkelstellungen arretierbar ist und daß die Bestrahlungskammer (35) durch einen zustellbaren Verschlußstopfen (40) verschließbar ist.

2. Bestrahlungsvorrichtung nach Anspruch 1,
**dadurch gekennzeichnet**,
daß das das zu bestrahlende Medium (Flüssigkeit 10) aufnehmende Behältnis (11; 21) mit einer zentrisch angeordneten Ausnehmung (13; 23) zur Aufnahme der Strahlungsquelle (41) versehen ist.

3. Bestrahlungsvorrichtung nach Anspruch 2,
**dadurch gekennzeichnet**,
daß das Behältnis (11) durch einen schraubenlinienförmig um eine die Strahlungsquelle (41) aufnehmenden hohlen Kern (13) gewickelten Schlauch (14) gebildet ist.

4. Bestrahlungsvorrichtung nach Anspruch 2,
**dadurch gekennzeichnet**,
daß das Behältnis (21) aus zwei mit Abstand ineinander angeordneten Rohren (22, 24) gebildet ist, und daß der durch diese eingeschlossene Ringraum durch Zwischenwände (25) in miteinander kommunizierende Strömungskanäle (26) unterteilt ist.

5. Bestrahlungsvorrichtung nach einem oder mehreren der Ansprüche 2 bis 4,
**dadurch gekennzeichnet**,
daß das Behältnis (11; 21) an eine Pumpvorrichtung (7), beispielsweise eine an der Bestrahlungsvorrichtung (1) angebrachte Schlauchpumpe, anschließbar ist.

6. Bestrahlungsvorrichtung nach einem oder mehreren der Ansprüche 1 bis 5,
**dadurch gekennzeichnet**,
daß der Schieber (51) mittels eines Hebelgestänges (55) in drei Betriebsstellungen (A, B, C) verstellbar und in diesen arretierbar ist.

7. Bestrahlungsvorrichtung nach Anspruch 6,
**dadurch gekennzeichnet**,
daß das Hebelgestänge (55) aus einem außerhalb des Gehäuses (31) angeordneten drehbar gelagerten Betätigungshebel (56), einem an dem Schieber (51) angebrachten Verstellhebel (58) und einem an diesen angelenkten Verbindungshebel (59) gebildet ist.

8. Bestrahlungsvorrichtung nach Anspruch 7,
**dadurch gekennzeichnet**,
daß dem Betätigungshebel (56) ein in Abhängigkeit von der Betriebsstellung des Verschlußstopfens (40) lösbares Verriegelungsglied (60) zugeordnet ist.

9. Bestrahlungsvorrichtung nach einem oder mehreren der Ansprüche 1 bis 8,
**dadurch gekennzeichnet**,
daß die Strahlungsquelle (41) mittels eines vorzugsweise motorisch antreibbaren Hubgliedes (43) in die Bestrahlungskammer (35) des Gehäuses (31) einführbar ist.

10. Bestrahlungsvorrichtung nach einem oder mehreren der Ansprüche 1 bis 9,
**dadurch gekennzeichnet**,
daß der Verschlußstopfen (40) mit Hilfe einer Brücke (73) an einem aus zwei Führungsschienen (72) bestehenden Traggestell (71) mittels eines Motors (74) höhenverstellbar geführt ist.

11. Bestrahlungsvorrichtung nach einem oder mehreren der Ansprüche 1 bis 10,
**dadurch gekennzeichnet**,
daß die Brücke (73) mit einem Schaltnocken (76) versehen ist, mittels dem das Verriegelungsglied (60) des dem Schieber (51) zugeordneten Hebelgestänges (55) lösbar ist.

12. Bestrahlungsvorrichtung nach einem oder mehreren der Ansprüche 1 bis 11,
**dadurch gekennzeichnet**,
daß das das zu bestrahlende Medium (Flüssigkeit 10) aufnehmende Behältnis (11; 21) an dem Verschlußstopfen (40), beispielsweise mittels einer gestuft ausgebildeten Schraube (78), lösbar befestigt ist.

13. Bestrahlungsvorrichtung nach einem oder mehreren der Ansprüche 1 bis 12,
**dadurch gekennzeichnet**,
daß der Verschlußstopfen (40) in eine fluchtend zu der Aufnahmekammer (35) des Behältnisses (11; 21) in das Gehäuse (31) eingearbeitete erweiterte Aufnahmeöffnung (36) einsetzbar ist.

14. Bestrahlungsvorrichtung nach Anspruch 13,
**dadurch gekennzeichnet**,
daß der Verschlußstopfen (40) mit einer die Aufnahmeöffnung (36) übergreifenden Abdeckung (77) versehen ist.

15. Bestrahlungsvorrichtung nach einem oder mehreren der Ansprüche 1 bis 14,
**dadurch gekennzeichnet**,
daß der Verschlußstopfen (40) vorzugsweise in einem Randbereich mit einer Freisparung (68) zur Durchführung von an das Behältnis (11; 12) anzuschließenden Leitungen (2, 3) versehen ist.

16. Bestrahlungsvorrichtung nach einem oder mehreren der Ansprüche 1 bis 15,
**dadurch gekennzeichnet**,
daß das Gehäuse (31) aus einem die Strahlungsquelle (41) aufnehmenden Unterteil (32), einem Zwischenstück (37) zur verdrehbaren Lagerung des Schiebers (51) und einem das Behältnis (11; 21) aufnehmenden Oberteil (34) zusammengesetzt ist.

17. Bestrahlungsvorrichtung nach einem oder mehreren der Ansprüche 1 bis 16,
**dadurch gekennzeichnet,**
daß das Gehäuse (31) mit einer geschlossenen Ummantelung (39) versehen ist.

18. Bestrahlungsvorrichtung nach einem oder mehreren der Ansprüche 1 bis 17,
**dadurch gekennzeichnet**,
daß an der Ummantelung (39) des Gehäuses (31) eine Anzeigeeinrichtung (31) angebracht ist, die mechanisch unmittelbar mit der Strahlungsquelle (41) oder deren Hubglied (43) verbunden ist.

## Claims

1. A device for irradiating an object, in particular a liquid or gaseous medium such as blood which is stored inside a container, with a radiation source for ionizing beams placed within a shielded housing in which it influences the medium to be irradiated,
**characterized in that,**
the housing (31) is preferably formed in the shape of a barrel with a central accommodation chamber (33) for the radiation source (41) at one end and, flush with the accommodation chamber (33), an irradiation chamber (35) on the other end for holding a container (11; 21) containing the medium (10) to be irradiated, that a slide valve (51) which is mounted so it can be turned or pushed, for example, is arranged between the accommodation chamber (33) and the irradiation chamber (35) with the aforementioned slide valve (51) having a passageway (52) for the radiation source (41) which can be inserted into the irradiation chamber (35) and being lockable in various angled settings, and that the irradiation chamber (35) can be closed by a plug (40) which can be moved towards it.

2. The irradiation device in accordance with Claim 1,
**characterized in that**,
the container (11; 21) holding the medium (liquid 10) to be irradiated has a central recess (13; 23) for accommodating the radiation source (41).

3. The irradiation device in accordance with Claim 2,
**characterized in that,**
the container (11) is formed by a helically shaped hose (14) wrapped around a hollow core (13) which accommodates the radiation source (41).

4. The irradiation device in accordance with Claim 2,
**characterized in that**,
the container (21) is formed by two tubes (22, 24) arranged one inside the other with a gap in between, and that the annular space enclosed by the tubes (22, 24) is sub-divided by intermediate walls (25) into intercommunicating flow channels (26).

5. The irradiation device in accordance with one or more of Claims 2 to 4,
**characterized in that,**
the container (11; 21) can be connected to a pump device (7), for example a hose pump attached to the irradiation device (1).

6. The irradiation device in accordance with one or more of Claims 1 to 5,
**characterized in that,**
the slide valve (51) can be set to three operating positions (A, B, C) in which it can be locked.

7. The irradiation device in accordance with Claim 6,
**characterized in that,**
the lever linkage (55) is formed from an actuation lever (56) mounted so as to rotate and located outside the housing (31), from an adjustment lever (58) attached to the slide valve (51) and from a connection lever (59) hinged on the adjustment lever (58).

8. The irradiation device in accordance with Claim 7,
**characterized in that,**
the actuation lever (56) is assigned to a locking element (60) which can be released depending on the operating position of the plug (40).

9. The irradiation device in accordance with one or more of Claims 1 to 8,
**characterized in that,**
the radiation source (41) can be inserted into the irradiation chamber (35) of the housing (31) by means of a stroke element (43) which can preferably be driven by a motor.

10. The irradiation device in accordance with one or more of Claims 1 to 9,
**characterized in that,**
the plug (40) is guided on a carrier frame (71) by means of a bridge (73) with the carrier frame (71) consisting of two guide rails (72), and the height of the plug (40) can be adjusted using a motor (74).

11. The irradiation device in accordance with one or more of Claims 1 to 10,
**characterized in that,**
the bridge (73) has a switching cam (76) by means of which the locking element (60) of the lever linkage (55) assigned to the slide valve (51) can be released.

12. The irradiation device in accordance with one or more of Claims 1 to 11,
**characterized in that,**
the container (11; 21) holding the medium (liquid 10) to be irradiated is fixed onto the plug (40) in a releasable connection, for example by means of a stepped screw (78).

13. The irradiation device in accordance with one or more of Claims 1 to 12,
**characterized in that,**
the plug (40) can be inserted in an expanded accommodation opening (36) worked into the housing (31) and flush with the accommodation chamber (35) of the container (11; 21).

14. The irradiation device in accordance with Claim 13,
**characterized in that,**
the plug (40) has a cover (77) extending beyond the accommodation opening (36).

15. The irradiation device in accordance with one or more of Claims 1 to 14,
**characterized in that,**
the plug (40) preferably has a notch (68) in its edge for passing through cables (2, 3) for connection to the container (11; 21).

16. The irradiation device in accordance with one or more of Claims 1 to 15,
**characterized in that**,
the housing (31) is composed of a base part (32) which accommodates the radiation source (41), of an adapter (37) for mounting the slide valve (51) so it can rotate and of an upper part (34) which accommodates the container (11; 21).

17. The irradiation device in accordance with one or more of Claims 1 to 16,
**characterized in that,**
the housing (31) has a closed jacket (39).

18. The irradiation device in accordance with one or more of Claims 1 to 17,
**characterized in that,**
a display device (31) is attached to the jacket (39) of the housing (31) and this display device (31) has a direct mechanical connection to the radiation source (41) or its stroke element (43).

## Revendications

1. Dispositif d'irradiation d'un objet, notamment d'un milieu liquide ou gazeux enfermé dans un réservoir, comme par exemple du sang, à l'aide d'une source d'irradiation à rayons ionisés qui est montée dans un boîtier blindé et qui, dans celui-ci, influe sur le milieu à soumettre à l'irradiation,
caractérisé en ce que
le boîtier (31) conçu de préférence sous forme d'un tonneau comporte d'un côté une chambre centrée (33) pour le logement de la source d'irradiation (41), et, alignée de l'autre côté de celle-ci, une chambre d'irradiation (35) destinée à recevoir un réservoir (11; 21) contenant le milieu (10) à soumettre à l'irradiation, qu'entre la chambre de logement (33) et la chambre d'irradiation (35), il est prévu un tiroir (51) p. ex. rotatif ou glissant, muni d'un passage (52) pour l'introduction de la source d'irradiation (41) dans la chambre d'irradiation (35) et positionné à divers angles, et que la chambre d'irradiation (35) se laisse obturer par un bouchon rapprochable (40).

2. Dispositif d'irradiation d'après la revendication 1,
caractérisé en ce que
le réservoir (11; 21) destiné à renfermer le milieu à soumettre à l'irradiation (liquide 10) est muni d'un évidement centré (13; 23) recevant la source d'irradiation (41).

3. Dispositif d'irradiation d'après la revendication 2,
caractérisé en ce que
le réservoir (11) est constitué par un tuyau flexible (14) enroulé sous forme hélicoïdale autour d'un noyau creux (13) recevant la source d'irradiation (41).

4. Dispositif d'irradiation d'après la revendication 2,
caractérisé en ce que
le réservoir (21) est constitué par deux tubes (22, 24) disposés l'un à l'intérieur de l'autre avec une certaine distance entre eux et que la chambre annulaire ainsi renfermée est subdivisée par des parois intermédiaires (25) en canaux d'écoulement (26) communiquant entre eux.

5. Dispositif d'irradiation d'après une ou plusieurs des revendications 2 à 4,
caractérisé en ce que
le réservoir (11; 21) se laisse brancher sur un dispositif de pompage (7), par exemple une pompe tubulaire prévue sur le dispositif d'irradiation (1).

6. Dispositif d'irradiation d'après une ou plusieurs des revendications 1 à 5,
caractérisé en ce que
le tiroir (51) se laisse amener par une tringlerie à leviers (55) en trois positions de service (A, B, C) où il se laisse bloquer.

7. Dispositif d'irradiation d'après la revendication 6,
caractérisé en ce que
la tringlerie à leviers (55) est formée par un levier d'actionnement (56) disposé à l'extérieur du boîtier (31) et logé en rotation, par un levier de réglage (58) monté sur le tiroir (51) et par un levier de raccordement (59) articulé sur ce dernier.

8. Dispositif d'irradiation d'après la revendication 7,
caractérisé en ce que,
au levier d'actionnement (56), il est assigné un organe de verrouillage (60) détachable en fonction de la position de service du bouchon (40).

9. Dispositif d'irradiation d'après une ou plusieurs des revendications 1 à 8,
caractérisé en ce que
la source d'irradiation (41) se laisse introduire dans la chambre d'irradiation (35) du boîtier (31) au moyen d'un membre de levage (43) entraîné de préférence par un moteur.

10. Dispositif d'irradiation d'après une ou plusieurs des revendications 1 à 9,
caractérisé en ce que,
à l'aide d'un pont (73) et d'un moteur (74), le bouchon (40) se laisse régler en hauteur sur un support (71) comprenant deux glissières de guidage (72).

11. Dispositif d'irradiation d'après une ou plusieurs des revendications 1 à 10,
caractérisé en ce que
le pont (73) est équipé d'une came de commande (76) qui permet de détacher l'organe de verrouillage (60) de la tringlerie à leviers (55) assignée au tiroir (51).

12. Dispositif d'irradiation d'après une ou plusieurs des revendications 1 à 11,
caractérisé en ce que
le réservoir (11; 21) destiné à renfermer le milieu à soumettre à l'irradiation (liquide 10) est fixé d'une manière détachable au bouchon (40), par exemple au moyen d'une vis étagée (78).

13. Dispositif d'irradiation d'après une ou plusieurs des revendications 1 à 12,
caractérisé en ce que
le bouchon (40) se laisse insérer dans une ouverture de logement élargie (36) pratiquée dans le boîtier (31) et alignée par rapport à la chambre de logement (33) du réservoir (11; 21).

14. Dispositif d'irradiation d'après la revendication 13,
caractérisé en ce que,
le bouchon (40) est muni d'un recouvrement (77) dépassant l'ouverture de logement (36).

15. Dispositif d'irradiation d'après une ou plusieurs des revendications 1 à 14,
caractérisé en ce que,
de préférence à un endroit extérieur, le bouchon (40) est muni d'un passage (68) destiné aux conduites (2, 3) à brancher sur le réservoir (11; 21).

16. Dispositif d'irradiation d'après une ou plusieurs des revendications 1 à 15,
caractérisé en ce que
le boîtier (31) consiste d'une partie inférieure (32) recevant la source d'irradiation (41), d'une pièce intermédiaire (37) pour le logement en rotation du tiroir (51) et d'une pièce supérieure (34) recevant le réservoir (11, 21).

17. Dispositif d'irradiation d'après une ou plusieurs des revendications 1 à 16,
caractérisé en ce que
le boîtier (31) est entouré d'un revêtement fermé (39).

18. Dispositif d'irradiation d'après une ou plusieurs des revendications 1 à 17,
caractérisé en ce que
sur le revêtement (39) du boîtier (31), il est prévu un indicateur (81) lié mécaniquement et directement à la source d'irradiation (41), ou à son membre de levage (43).
